# FASCICULE DE BREVET EUROPEEN

(11) **EP 2 285 427 B1**
(45) Date de publication et mention de la délivrance du brevet: **29.02.2012**
(21) Numéro de dépôt: 09745910.1
(22) Date de dépôt: 29.04.2009
(51) Int. Cl.: A61L 27/50, A61L 27/18, A61F 2/16

(54) **MATÉRIAU POLYMÈRE ACRYLIQUE, HYDROPHOBE POUR LENTILLE INTRAOCULAIRE, À SOUPLESSE AMÉLIORÉE PAR L'UTILISATION D'UN AGENT DE TRANSFERT.**
HYDROPHOBES ACRYLPOLYMERMATERIAL FÜR EINE INTRAOKULARLINSE MIT ERHÖHTER FLEXIBILITÄT DURCH DEN EINSATZ EINES ÜBERTRAGUNGSMEDIUMS
HYDROPHOBIC ACRYLIC POLYMER MATERIAL FOR AN INTRAOCULAR LENS, HAVING AN ENHANCED FLEXIBILITY DUE TO THE USE OF A TRANSFER AGENT

(30) Priorité: 30.04.2008 FR 0802427
(43) Date de publication de la demande: 23.02.2011
(73) Titulaire: Acrylian, 67100 Strasbourg (FR)
(72) Inventeur: TERRISSE, Jean, 67100 Strasbourg (FR)
(74) Mandataire: Metz, Paul
(86) Numéro de dépôt international: PCT/FR2009/000505
(87) Numéro de publication internationale: WO 2009/138591

(56) Documents cités:
- WO-A-01/05578
- WO-A-01/18079
- US-B1- 6 326 448

## Description

La présente invention concerne un nouveau matériau polymère acrylique, hydrophobe, à souplesse améliorée par l'ajout d'un agent de transfert, ce matériau étant parfaitement adapté pour la réalisation de lentilles intraoculaires.

L'invention concerne également un procédé de fabrication d'un tel matériau polymère, ainsi que des lentilles intraoculaires réalisées à partir de ce nouveau matériau polymère.

Les lentilles intraoculaires sont des implants ou prothèses ophtalmologiques qui sont mises en place chirurgicalement dans l'oeil de patients souffrant par exemple de cataracte, en remplacement de leur cristallin défaillant.

Lors de cette intervention chirurgicale, le chirurgien commence par retirer le cristallin naturel malade du patient. Pour cela, il pratique une petite incision dans la cornée du patient, à travers laquelle il insère un outil appelé phacoémulsificateur, dont l'extrémité vient piquer la paroi antérieure du sac cristallinien et la traverse.

Cet outil permet de fragmenter le cristallin par émission d'ultrasons, d'injecter du sérum physiologique pour mettre les fragments en solution et d'aspirer simultanément la solution contenant les fragments de cristallin.

Le chirurgien, après y avoir injecté un lubrifiant de type gel de hyaluronate, met en place dans le sac cristallinien la lentille intraoculaire devant remplacer le cristallin.

Cette lentilles comporte classiquement une partie optique correctrice, dont la correction varie selon les cas de 10 à 30 dioptries. Cette partie optique est sensiblement en forme de disque et présente une section généralement biconvexe asymétrique. Elle doit être positionnée de manière centrée par rapport à l'axe optique de l'oeil.

De cette partie optique centrale s'étendent des prolongements latéraux appelés haptiques, généralement au nombre de deux ou de quatre et pouvant être de formes variées. Le rôle de ces haptiques est de tendre les parois du sac cristallinien et d'assurer un positionnement correct de la lentille par rapport à l'axe optique de l'oeil.

La lentille intraoculaire est implantée dans l'oeil du patient par l'incision de la cornée ayant préalablement servi à retirer le cristallin, au moyen d'un injecteur comportant un tube d'injection de diamètre extérieur inférieur à celui de l'incision et dont l'extrémité ouverte est introduite à travers cette incision.

Afin que l'intervention soit la moins traumatisante possible pour le patient et pour éviter le développement d'un astigmatisme postopératoire, l'incision réalisée dans la cornée doit être la plus petite possible.

La largeur de ces incisions a considérablement diminuée au cours des dernières années. On pratique actuellement des incisions de l'ordre de 2,5 mm de long et l'on souhaite atteindre bientôt une largeur de 2,2 mm, voire 2 mm dans le futur.

La lentille intraoculaire, d'un diamètre nettement supérieur, doit donc être préalablement placée dans une configuration repliée d'insertion permettant son passage à travers cette incision de très faible longueur.

Pour cela, la lentilles est placée, avec une solution aqueuse d'acide hyaluronique par exemple, dans la chambre de chargement d'une cartouche d'injection qui provoque à sa fermeture l'enroulement de la lentille sur elle-même. Une fois la cartouche d'injection montée dans l'injecteur, un piston vient pousser l'extrémité arrière de la lentille dans le tube d'injection de injecteur, la comprimant encore ainsi que ses haptiques et provoquant son expulsion en dehors de l'injecteur dans le sac cristallinien de l'oeil du patient.

Une fois libérée dans le sac cristallinien, la lentille intraoculaire doit se déployer rapidement pour pouvoir se positionner correctement et être capable de remplir sa fonction de correction optique de manière satisfaisante.

Pour que la lentille puisse être posée sans problème, le matériau doit être suffisamment souple pour pouvoir être plié et enroulé sur lui-même. Il doit résister à l'étirement et à la pression de poussée sans se rompre, ni casser le tube d'injection, de manière à passer par un orifice d'éjection de diamètre extrêmement réduit, de l'ordre de 1,5 mm ou même moins.

Enfin, une fois dans l'oeil du patient, la lentilles intraoculaire doit être capable de se déployer toute seule, sans rester collée sur elle-même et en un temps relativement court, afin de se positionner correctement dans le sac cristallinien et de retrouver ses caractéristiques optiques.

Dans l'art antérieur, on a tenté de diminuer la taille des lentilles intraoculaires de manière à pouvoir les introduire plus facilement à travers une incision de plus en plus petite. Pour conserver les propriétés optiques de ces lentilles, on a dû rechercher des matériaux d'indice optique de plus en plus élevé. Cependant, on a alors été confronté à des problèmes d'éblouissement liés à des réflexions multiples de la lumière entre la rétine et la lentille.

WO 01/05578 A décrit un matériau polymère acrylique, hydrophobe, destiné à la réalisation d'implants ophtalmologiques obtenu par polymérisation radicalaire par transfert d'atome à partir d'un mélange de monomères acryliques, comprenant comme agent de transfert un produit halogéné, comprenant également un initiateur mais pas de composés réticulants.

US 6,326,448 B1 décrit un matériau polymère pour lentilles intraoculaires, obtenu par copolymérisation d'un (meth)acrylate, préférablement le 2-phénoxyéthyl acrylate (POEA), d'un monomère hydrophile, préférablement le 2-hydroxyéthylméthacrylate (HEMA), d'un alkyl(méth)acrylate et d'un monomère réticulable, préférablement le diacrylate de butanediol (BDDA). Le matériau obtenu est flexible, possède un indice de réfraction élevé et ne scintille pas.

WO 01/18079 A décrit un matériau polymère pour lentilles intraoculaires, obtenu par copolymérisation d'un monomère hydrophobe, préférablement le 2-phénoxyéthyl méthacrylate (POEMA), d'un monomère hydrophile, préférablement le 2-hydroxyéthylméthacrylate (HEMA), d'un diméthacrylate d'éthylène glycol et d'un initiateur. Le matériau obtenu est flexible, possède un indice de réfraction d'au moins 1.5 et une température de transition vitreuse inférieure à 15°C.

Une autre voie a consisté à utiliser un matériau plus déformable pour réaliser des lentilles de même taille et d'indice constant.

Les matières plastiques utilisées pour cela dans l'art antérieur peuvent être classées en deux grandes catégories : les matières plastiques dites « hydrophiles » et celles dites « hydrophobes ».

Les matières plastiques « hydrophiles » sont les plus déformables. Leur taille et leur souplesse dépendent de la quantité d'eau absorbée. Elles sont dures et compactes en absence d'eau, ce qui permet de les usiner facilement à température ambiante. Lorsqu'elles sont hydratées, elles se dilatent et deviennent molles et souples, ce qui leur permet d'être implantées dans l'oeil.

Cependant, ces lentilles dites **«** hydrophiles » qui contiennent près de 25% d'eau à la température de l'oeil, sont en équilibre avec l'eau de l'oeil dans lequel elles sont implantées. Or, comme il est difficile de débarrasser entièrement les lentilles hydrophiles de leurs impuretés de synthèse et résidus de fabrication, des produits provenant de la lentilles peuvent diffuser dans l'oeil et provoquer des inflammations voire dans certains cas désordres sévères.

Les matières plastiques de la deuxième catégorie, dites « hydrophobes », présentent des caractéristiques propres qui ne dépendent pas de la quantité d'eau absorbée. Elles peuvent être facilement purfiées et débarrassées des produits résiduels à la fabrication insolubles dans l'eau.

Il s'agit par exemple de polymères acryliques ou à base de silicone.

La souplesse de ces matériaux dépend de la température à laquelle ils se trouvent. Ils présentent une température de transition vitreuse (Tg) en dessous de laquelle ils sont durs et peuvent être usinés et au-dessus de laquelle ils deviennent souples, déformables et élastiques.

Pour la réalisation de lentilles intraoculaires, on doit choisir un matériau présentant une température de transition vitreuse suffisamment basse pour que la lentille résultante soit assez souple pour être roulée et étirée à la température d'une salle d'opération, soit environ 18 à 20°C.

L'invention se place dans le cadre de ces matières plastiques dites « hydrophobes » et visent plus spécifiquement les polymères acryliques.

Le problème bien connu de ces matériaux hydrophobes est que plus ils sont souples et déformables et plus ils deviennent collants.

De ce fait, les lentilles intraoculaires peuvent avoir du mal à se déployer correctement lorsqu'elles sont implantées dans l'oeil du patient. En particulier, les haptiques restent très souvent collées à la partie optique de la lentille.

Ce défaut se produit quelle que soit la forme géométrique des haptiques. Il est aggravé par le fait que pour diminuer l'encombrement global des lentilles afin de pouvoir les faire passer par une incision de taille minimale, on réduit le plus possible l'épaisseur de la zone médiane de la lentille, inactive optiquement, et des haptiques qui la prolongent.

L'épaisseur étant réduite au niveau de la zone de rattachement des haptiques formant charnière, la force de ressort de la matière, servant normalement à déplier les haptiques, est nettement diminuée et ne suffit plus à vaincre la force d'adhérence de la matière collante et à décoller les haptiques de la partie optique de la lentilles.

Le chirurgien doit alors tenter de les décoller manuellement à l'aide d'un petit outil qu'il passe à travers l'incision à la place de l'injecteur. Cette opération, risquée et particulièrement délicate, se solde le plus souvent par un échec.

Pour résoudre ce problème technique, on a proposé dans l'art antérieur de traiter la surface des lentilles, postérieurement à leur fabrication, pour les rendre plus glissantes et moins collantes. La demande de brevet WO 94/25510 propose par exemple d'exposer pour cela leur surface à un plasma.

L'invention offre une solution différente à ce problème en fournissant un nouveau matériau polymère acrylique, hydrophobe, souple, destiné à la réalisation d'implants ophtalmologiques et plus particulièrement de lentilles intraoculaires.

Ce matériau est obtenu par polymérisation radicalaire à partir d'un mélange de monomères acryliques ou d'un mélange de monomères acryliques et méthacryliques. Selon l'invention, ce mélange comprend en outre au moins un agent de transfert, notamment un thiol contenant préférentiellement de quatre à dix-sept atomes de carbone, ou un produit halogéné de préférence chloré.

Des lentilles intraoculaires à implanter chirurgicalement dans le sac cristallinien, en remplacement du cristallin naturel, peuvent avantageusement être réalisées à partir d'un tel matériau polymère acrylique.

L'invention fournit également un procédé de fabrication d'un tel matériau polymère acrylique par polymérisation radicalaire à partir d'un mélange de monomères acryliques ou d'un mélange de monomères acryliques et méthacryliques, selon lequel on ajoute un agent de transfert aux monomères avant leur polymérisation.

Pour démarrer le processus de polymérisation de ce matériau, on peut ajouter au mélange un ou plusieurs composés initiateurs.

Selon un mode de réalisation préférentiel de l'invention, le mélange de monomères peut avantageusement contenir au moins :
- un arylalcoxy-acrylate ou un arylalcoxy-méthacrylate ;
- un alkylacrylate ;
- un acrylate hydroxylé ;
- un méthacrylate hydroxylé ;
- un diacrylate de diol ; et
- un diméthacrylate de diol.

Grâce à l'action de l'agent de transfert, le matériau polymère acrylique selon l'invention est, de façon surprenante, particulièrement souple et déformable à température ambiante et présente une grande aptitude à la déformation sans rupture aux températures de pose et d'utilisation de la lentille, c'est-à-dire entre 18 et 35°C. Il peut être facilement roulé et étiré sans problème pour être implanté dans l'oeil du patient.

Les présents inventeurs sont parvenus ainsi à faire passer sans dommage une lentille réalisée à partir d'un matériau selon l'invention, à travers une incision de 1,5 mm de largeur, ce qui correspond à une largeur très nettement inférieure aux 2,5 mm pratiqués actuellement et même nettement inférieure aux 2 mm espérés par la profession, et ce qui représente un progrès considérable et surprenant par rapport à l'état de la technique.

Le matériau selon l'invention est en outre non collant sur lui-même en présence d'eau. Il peut donc se déployer facilement et entièrement une fois en position dans l'oeil du patient et résout ainsi de façon satisfaisante le problème des haptiques restant collées à la partie optique des lentilles intraoculaires.

La force de ressort nécessaire au niveau de la charnière pour déplier les haptiques étant beaucoup plus faible puisqu'il y a moins d'adhérence, l'épaisseur de la zone médiane de la lentille et de ses haptiques peut avantageusement être réduite pour diminuer l'encombrement global de la lentille, sans conséquence néfaste pour son déploiement à la pose.

D'autres caractéristiques et avantages de l'invention apparaîtront à la lecture de la description détaillée qui va suivre.

Pour faciliter la bonne compréhension du lecteur, cette description est accompagnée à titre d'exemple des dessins annexés suivants :
- la figure 1 est un premier exemple de lentille intraoculaire pouvant être réalisée à partir d'un matériau selon l'invention ;
- la figure 2 est un deuxième exemple de lentille intraoculaire pouvant être réalisée à partir d'un matériau selon l'invention ;
- la figure 3 est un dessin schématique illustrant l'implantation dans l'oeil d'un patient d'une lentille intraoculaire réalisée à partir d'un matériau selon l'invention.

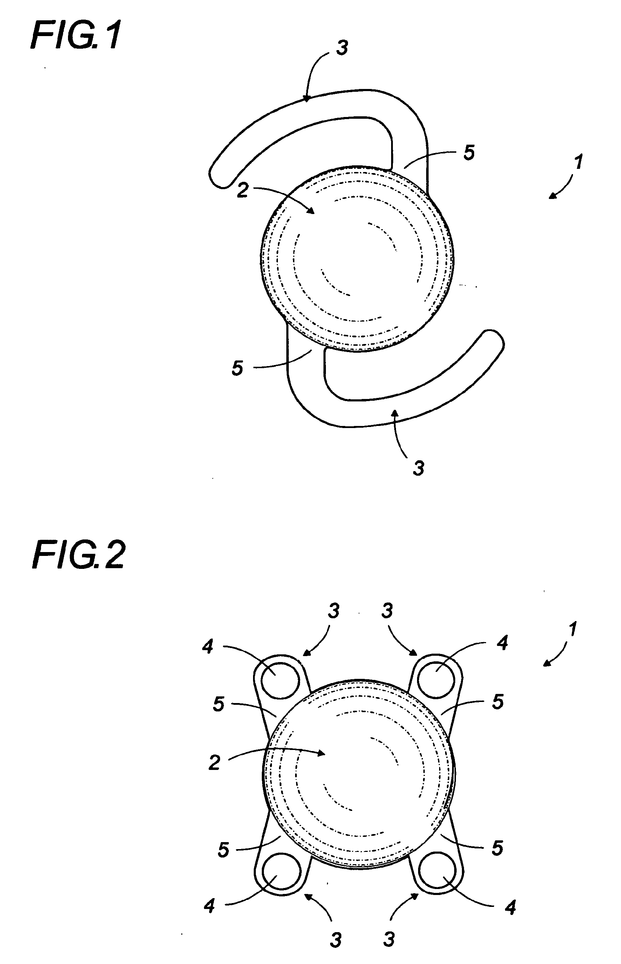

Sur les figures 1 et 2, on a représenté deux exemples classiques de lentille intraoculaire 1 pouvant être réalisée à partir d'un matériau polymère acrylique selon finven6on.

Ces lentilles 1 comportent une partie optique 2 centrale, sensiblement en forme de disque et à profil bi-convexe.

De cette partie optique 2 s'étendent des prolongements latéraux appelés haptiques 3.

Sur la figure 1, ces haptiques 3 sont au nombre de deux. Elles sont disposées de manière diamétralement opposée et présentent une forme de bras recourbé allant chacun dans un sens opposé.

La lentille de la figure 2 comporte quatre haptiques 3 en forme d'anneau percé d'un orifice central 4. Ces haptiques 3 sont régulièrement réparties sur le pourtour de la partie optique 2.

Sur les exemples représentés, les haptiques 3 sont réalisées d'une pièce avec la partie optique 2 de la lentille 1. Ce type de lentilles est appelé « lentille monobloc ». Le matériau selon l'invention est parfaitement adapté à la réalisation de telles lentilles.

Les haptiques 3 sont reliées à la partie optique centrale 2 par une zone de jonction 5 formant charnière qui génère un effet de ressort par rappel élastique de la matière pour déplier la lentille lors de son implantation dans l'oeil d'un patient.

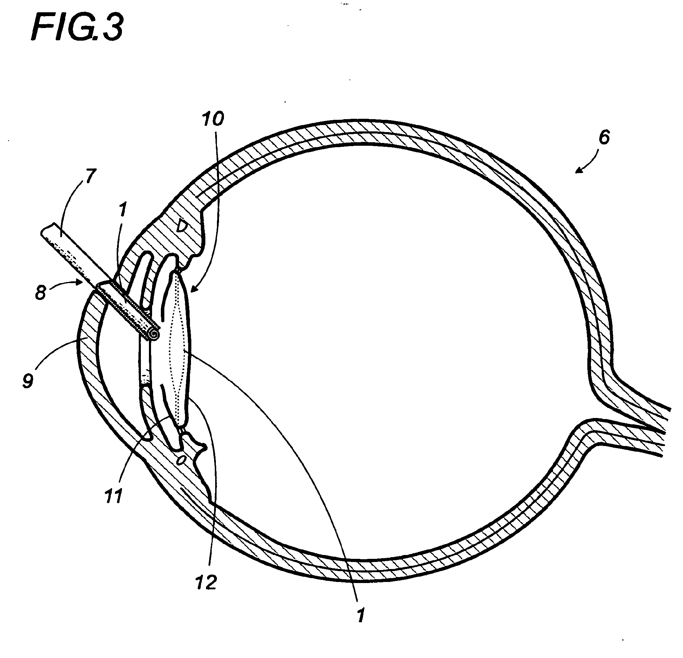

La figure 3 illustre schématiquement l'intervention d'implantation d'une lentille intraoculaire 1 dans l'oeil 6 d'un patient.

Comme expliqué dans la partie introductive, la lentilles 1 est enroulée sur elle-même à l'intérieur d'un injecteur 7 dont l'extrémité est introduite à travers une incision 8 de petite taille, préalablement réalisée dans la cornée 9 du patient.

L'extrémité de l'injecteur 7 pénètre ensuite dans le sac cristallinien 10 préalablement vidé du cristallin naturel.

La lentille intraoculaire 1 est alors éjectée dans le sac cristallinien 10 où elle se déploie et se positionne grâce à ses haptiques 3 entre la paroi antérieure 11 et la paroi postérieure 12 du sac. La lentille intraoculaire 1 est maintenue par ses haptiques 3 en position d'utilisation, comme représenté en traits pointillés sur la figure 3.

Le matériau selon l'invention est particulièrement adapté à la réalisation de telles lentilles intraoculaires 1.

Il s'agit d'un polymère acrylique obtenu par polymérisation radicalaire à partir d'un mélange de monomères acryliques et/ou méthacryliques et qui constitue un système acrylique réticulé.

Selon l'invention, le mélange initial de monomères contient en outre un ou plusieurs agent(s) de transfert.

De façon surprenante, les présents inventeurs ont constaté que l'ajout d'une petite quantité d'agent de transfert à ces systèmes acryliques réticulés permettait de réduire simultanément et considérablement deux propriétés particulièrement gênantes et bien connues de ces polymères acryliques : d'une part la fragilité et le caractère cassant des polymères fortement réticulés qui se traduit par un allongement à la rupture inversement proportionnel au taux de réticulation, et d'autre part le caractère collant de ces polymères qui augmente avec la longueur de chaîne entre les noeuds de réticulation et devient particulièrement gênant pour les systèmes peu réticulés.

Ces deux propriétés agissent en sens contraire : Plus on augmente le taux de réticulation et moins le polymère résultant est collant, mais plus il est fragile et casse avec une déformation limitée à des valeurs comprises entre 60 et 100%.

Les inventeurs ont remarqué que l'agent de transfert augmente avantageusement l'aptitude à la déformation sans rupture du matériau. L'ajout au mélange d'un agent de transfert permet d'avoir un taux de réticulation élevé et donc un polymère résultant peu collant, tout en gardant de façon surprenante un allongement à la rupture important.

Cet agent de transfert arrête localement la polymérisation en transférant le radical d'un monomère à un autre. La formation du maillage tridimensionnel est ainsi localement interrompue et l'on obtient à ce niveau une maille coupée avec une chaîne pendante courte reliée au réseau mais dont l'autre extrémité reste libre. L'agent de transfert permet ainsi d'obtenir un maillage plus lâche capable de s'étirer davantage sans rupture pour un taux de réticulation élevé.

L'agent de transfert peut être un produit halogéné, par exemple chloré, ou plus préférentiellement un composé de la famille des thiols.

Il s'agit de préférence d'un thiol contenant de quatre à dix-sept atomes de carbone, par exemple le butane thiol, l'octane thiol ou le dodécane thiol, ces deux derniers composés étant préférés car plus faciles à doser et plus stables en composition.

Bien entendu, la composition peut contenir plusieurs agents de transfert, préférentiellement choisis parmi les composés cités ci-dessus.

Avantageusement, une très petite quantité d'agent de transfert est nécessaire à l'obtention de ce résultat. Le mélange initial contient ainsi préférentiellement entre 0,03% et 0,1% en masse d'agent de transfert, plus préférentiellement encore entre 0,04% et 0,07% d'agent de transfert, 0,05% étant une valeur préférée pour le butane thiol.

Cet ou ces agent(s) de transfert ont pour fonction de modifier la structure du réseau macromoléculaire de manière à en augmenter considérablement la déformabilité avant rupture et à ralentir la vitesse de propagation des fissures sous contrainte.

Ils jouent sur les propriétés mécaniques du réseau sans en modifier la chimie. Ils permettent ainsi d'obtenir, à taux de réticulation constant, une déformabilité beaucoup plus grande du réseau constitué qui se traduit par un ralentissement considérable de l'apparition et de la propagation des fissures comme le prouvent les deux séries de tests décrits ci-dessous.

Afin de mettre en évidence l'action de l'agent de transfert, les tests ont été effectués sur plusieurs matériaux polymères réalisés avec ou sans agent de transfert, et avec un agent de transfert utilisé en quantité variable.

Dans chaque série, trois polymères différents ont été obtenus à partir d'un même mélange initial de monomères et d'initiateur et avec une quantité variable d'agent de transfert. Les quantités sont exprimées en pourcentage massique de monomères.

### Polymères de la série A : A₀, A_{0,05} et A_{0,08}

### MONOMÈRES:

- phénoxy-éthylacrylate 83%
- acrylate d'hexyle 10%
- méthacrylate d'hydroxy-éthyle 5,5%
- diacrylate d'éthylène glycol 1,5%

### COMPOSÉ INITIATEUR :

- peroxyde 0,5%

| AGENT DE TRANSFERT: | **A₀** | **A_{0,05}** | **A_{0,08}** |
|---|---|---|---|
| - butane thiol | 0% | 0,05% | 0,08% |

### Polymères de la série B : B₀, B_{0,04} et B_{0,07}

### MONOMÈRES :

- 2-éthoxy-éthylméthacrylate 73%
- acrylate de butyle 17%
- méthacrylate d'hydroxy-éthyle 8%
- diméthacrylate d'éthylène glycol 2%

### COMPOSÉ INITIATEUR :

- peroxyde 0,8%

| AGENT DE TRANSFERT: | Polymère: | **B₀** | **B_{0,04}** | **B_{0,07}** |
|---|---|---|---|---|
| - butane thiol | | 0% | 0,04% | 0,07% |

### Polymères de la série C : C₀, C_{0,05} et C_{0,1}

### MONOMÈRES :

- 2-éthoxy-éthylméthacrylate 61,5%
- acrylate de lauryle 37%
- diméthacrylate d'éthylène glycol 1,5%

### COMPOSÉ INITIATEUR :

- peroxyde 0,5%

| AGENT DE TRANSFERT: | Polymère: | **C₀** | **C_{0,05}** | **C_{0,1}** |
|---|---|---|---|---|
| - butane thiol | | 0% | 0,05% | 0,1% |

### Polymères de la série D : D₀, D_{0,04} et D_{0,08}

### MONOMÈRES :

- phénoxy-éthylacrylate 73%
- méthacrylate d'hydroxy-éthyle 11 %
- acrylate de butyle 15%
- diméthacrylate de diéthylène glycol 1%

### COMPOSÉ INITIATEUR :

- peroxyde 0,5%

| AGENT DE TRANSFERT: | Polymère: | **D₀** | **D_{0,04}** | **D_{0,08}** |
|---|---|---|---|---|
| - butane thiol | | 0% | 0,04% | 0,08% |

On a formé avec les différents matériaux testés des feuilles de 3 mm d'épaisseur dans lesquelles on a découpé des disques de 16 mm de diamètre. Pour chacun des matériaux testés, on a réalisé un lot d'une dizaine d'échantillons, on a effectué les mesures sur chaque échantillon et on a calculé un résultat moyen pour le lot.

La première série de tests avait pour objectif de comparer la résistance à la fissuration spontanée de ces matériaux en cas de déformation.

Pour cela, on a replié les disques sur eux-mêmes selon un pliage diamétral à 180°. Les disques repliés ont été placés entre deux plaques de verre, l'ensemble étant maintenu en place par une pince. Ces montages ont été plongés dans de l'eau à 35°C pendant deux minutes afin de garantir une température expérimentale constante et uniforme pour les différents échantillons. On a alors compté le nombre d'échantillons sur lesquels une fissure été apparue spontanément au niveau de la pliure. Ce nombre a ensuite été converti en taux de fissuration en moins de deux minutes par lot d'échantillons, ce taux étant exprimé en pourcentage.

La deuxième série de tests avait pour objectif de comparer la vitesse de propagation d'une fissure provoquée dans ces différents matériaux.

Pour cela, on a comme précédemment replié les disques sur eux-mêmes selon un pliage diamétral à 180° et placés ceux-ci entre deux plaques de verre, l'ensemble étant maintenu par une pince. On a alors marqué chaque disque par deux repères situés sur la ligne de pliage, distants chacun de 3 mm de l'un des bords. Les montages ont ensuite été plongés dans de l'eau à 35°C.

Après un temps d'attente de deux minutes, permettant au montage d'atteindre la température expérimentale d'équilibre, on a amorcé la propagation d'une fissure en réalisant sur chaque échantillon une entaille au scalpel sur l'un des bords de la pliure. La propagation de la fissure le long de la pliure a été observée au moyen d'une caméra. L'analyse des films a permis de mesurer pour chaque échantillon le temps nécessaire à la fissure pour se propager du premier repère au second repère. Les mesures, réalisées d'un repère à l'autre, sont ainsi indépendantes des effets de bord. Le temps de propagation mesuré a ensuite été converti en vitesse de propagation de fissure exprimée en millimètre par seconde.

Afin de permettre une analyse comparative, les résultats de ces deux tests pour chaque série de polymères ont été regroupés dans les tableaux ci-dessous.

| Polymères de la série A : | | | |
|---|---|---|---|
| | **A₀** | **A_{0,05}** | **A_{0,08}** |
| Test n°1 : taux de fissuration en moins de 2 minutes | 100% | 0% | 30% |
| Test n°2 : vitesse de propagation de fissure | 2mm/s | 0,2mm/s | 0,7mm/s |

| Polymères de la série B : | | | |
|---|---|---|---|
| | **B₀** | **B_{0,04}** | **B_{0,04}** |
| Test n°1 : taux de fissuration en moins de 2 minutes | 100% | 0% | 0% |
| Test n°2 : vitesse de propagation de fissure | 3mmls | 0,8mm/s | 1,2mm/s |

| Polymères de la série C : - | | | |
|---|---|---|---|
| | **C₀** | **C_{0,05}** | **C_{0,1}** |
| Test n°1 : taux de fissuration en moins de 2 minutes | 100% | 0% | 0% |
| Test n°2 : vitesse de propagation de fissure | 2,5mm/s | 1,5mm/s | 2mm/s |

| Polymères de la série D : | | | |
|---|---|---|---|
| | **D₀** | **D_{0,04}** | **D_{0,08}** |
| Test n°1 : taux de fissuration en moins de 2 minutes | 80% | 0% | 0% |
| Test n°2 : vitesse de propagation de fissure | 1,3mm/s | 0,3mm/s | 0,8mm/s |

On peut ainsi constater que l'ajout d'une petite quantité d'agent de transfert permet d'améliorer considérablement la résistance à la fissuration spontanée et de réduire fortement la vitesse de propagation des fissures provoquées. Ce résultat est indépendant de la nature des monomères acrylates et/ou méthacrylates utilisés.

D'après ces tests, des résultats optimums semblent être obtenus avec une teneur en butane thiol voisine de 0,04 à 0,05%.

Grâce à l'ajout d'un agent de transfert, on peut donc augmenter la réticulation du polymère acrylique final afin de réduire son caractère collant, sans rendre celui-ci fragile et cassant. On obtient avantageusement un matériau polymère acrylique souple et non collant particulièrement bien adapté à la réalisation de lentilles intraoculaires qui peuvent ainsi être déformées et étirées sans dommage pour être insérées à travers de très petites incisions et qui se déploient ensuite sans rester collée une fois mise en place dans l'oeil du patient.

L'ajout d'un agent de transfert procure encore un autre avantage lors de la fabrication des lentilles intraoculaires, qui est de rendre le polymère acrylique plus facilement usinable en empêchant les copeaux formés de coller à la lentille en cours de fabrication.

En plus des monomères et de l'agent de transfert, le mélange initial peut contenir un certain nombre de composés supplémentaires de nature différente.

Il comprend ainsi par exemple un composé initiateur qui sert à amorcer la réaction de polymérisation en créant des sites actifs sur les monomères. Ce composé permet de régler la cinétique de la réaction de polymérisation.

Le mélange peut contenir plusieurs composés initiateurs et de préférence deux composés initiateurs de réactivité très différente, par exemple l'un dix à vingt fois plus rapide que le second.

Les peroxydes d'alkyle sont des exemples de composés initiateurs utilisables, avec préférentiellement le diperoxyde de lauroyl communément appelé peroxyde de lauroyl, qui, très réactif dès 80°C, est le moins dangereux de cette famille. On peut lui ajouter éventuellement un second initiateur plus stable tel que par exemple le 1,1-di-ter-butylperoxycyclohexane ou un équivalent sensiblement de même réactivité.

Ce ou ces composés sont ajoutés au mélange en très petite quantité, le mélange comprenant par exemple entre 0,3 et 1% en masse de composé initiateur.

Le mélange peut également contenir par exemple un ou plusieurs agent(s) anti-ultraviolets, un ou plusieurs colorant(s) polymérisable(s) ou non polymérisable(s), ou tout autre constituant imaginable par l'homme du métier, de fonction quelconque, compatible avec la réalisation de lentilles intraoculaires.

La teneur du mélange en agent(s) anti-ultraviolets et/ou en colorant(s) est préférentiellement comprise entre 0.1 et 1% en masse.

Pour obtenir le polymère acrylique selon l'invention, on polymérise par voie radicalaire un mélange de monomères acryliques et/ou méthacryliques contenant en outre au moins un agent de transfert.

A titre d'exemple non limitatif, on va décrire ci-dessous un mélange de monomères particuliers qui permet d'obtenir selon l'invention un matériau polymère particulièrement avantageux. Il est évident que l'homme du métier pourra imaginer de nombreux autres mélanges à base de monomères acryliques et/ou méthacryliques différents sans sortir du cadre de l'invention défini par les revendications.

Selon ce mode de réalisation préférentiel, le mélange de monomères contient au moins :
- un arylalcoxy-acrylate ou un arylalcoxy-méthacrylate ;
- un alkylacrylate ;
- un acrylate hydroxylé ;
- un méthacrylate hydroxylé ;
- un diacrylate de diol ; et
- un diméthacrylate de diol.

L'utilisation d'un arylalcoxy-acrylate ou d'un arylalcoxy-méthacrylate permet d'obtenir un polymère final d'indice optique élevé.

On peut utiliser comme arylalcoxy-acrylate ou arylalcoxy-méthacrylate un composé choisi pour l'arylalcoxy-acrylate parmi le 2-phénoxy-éthylacrylate, le 2-phénoxy-2-éthoxy-éthylacrylate, le 2-phénoxy-2-éthoxy-2-éthoxy-éthylacrylate et ses oligomères supérieurs, et pour l'arylalcoxy-méthacrylate parmi le 2-phénoxy-éthylméthacrylate, le 2-phénoxy-2-éthoxy-éthylméthacrylate, le 2-phénoxy-2-éthoxy-2-éthoxy-éthylméthacrylate et ses oligomères supérieurs.

On peut citer à titre d'exemple préférentiel le 2-phénoxy-éthylacrylate, les arylalcoxy-acrylates étant en général préférés aux arylalcoxy-méthacrylates car ils présentent une température de transition vitreuse nettement inférieure.

Le mélange initial avant polymérisation comprend préférentiellement entre 45 et 89% en masse d'arylalcoxy-acrylate ou d'arylalcoxy-méthacrylate. De préférence, il contient entre 66 et 75% en masse d'arylalcoxy-acrylate ou d'arylalcoxy-méthacrylate, avec une valeur préférée de l'ordre de 74%.

On ajoute au mélange au moins un alkylacrylate dans le but de baisser la température de transition vitreuse du polymère résultant. On peut donc utiliser pour cela tous les alkylacrylates qui, une fois polymérisés, présentent une faible température de transition vitreuse et ne sont pas trop collants.

Pour des raisons pratiques, on se limite de préférence aux alkylacrylates présentant une température d'ébullition supérieure à 100°C afin d'éviter les problèmes de bulles.

L'alkylacrylate utilisé comporte préférentiellement une chaîne alkyle possédant de 4 à 6 atomes de carbone, l'acrylate de butyle pouvant avantageusement être choisi parmi ceux-ci.

Le mélange initial avant polymérisation comprend préférentiellement entre 5 et 20% en masse d'alkylacrylate, plus préférentiellement entre 10 et 15%, 10% étant une valeur préférée.

Pour diminuer le caractère collant du matériau en présence d'eau, le polymère - final doit avoir une quantité suffisante de fonctions hydroxyles en surface. L'eau forme ainsi un film continu à la surface du matériau qui ne colle plus sur lui-même dès lors qu'il présente une tension de surface supérieure à 0,04 N/m (40 dynes/cm).

On ajoute ainsi au mélange des monomères hydroxylés : un acrylate hydroxylé et un méthacrylate hydroxylé, qui augmentent la tension de surface et l'affinité de la surface avec l'eau du polymère résultant.

Pour être acceptables ces monomères ne doivent pas présenter, à l'état polymérisé, une température de transition vitreuse trop élevée, c'est-à-dire supérieure à 0°C.

L'acrylate hydroxylé utilisé est par exemple un mono-acrylate de dihydroxy-alkyle ou de dihydroxy-éthoxy-alkyle dont la chaîne alkyle du glycol comporte de 2 à 6 atomes de carbone. On peut citer par exemple l'acrylate d'hydroxy-éthyle, l'acrylate d'hydroxybutyle dit acrylate de butanediol, l'acrylate d'hexanediol, le monoacrylate de diéthyléne glycol et le monoacrylate de triéthylène glycol.

Le méthacrylate hydroxylé utilisé est préférentiellement un monométhacrylate de dihydroxy-alkyle ou de dihydroxy-éthoxy-alkyle dont la chaîne alkyle du glycol comporte de 2 à 6 atomes de carbone. Il s'agit par exemple du méthacrylate d'hydroxy-éthyle, du monométhacrylate de butanediol, du monométhacrylate d'hexanediol, du monométhacrylate de diéthylène glycol ou du monométhacrylate de triéthylène glycol.

La proportion de ces monomères hydroxylés dans le mélange avant polymérisation doit être suffisante pour que le matériau résultant présente une tension de surface convenable et ne blanchisse pas au contact prolongé de l'eau à 35°C. Elle ne doit cependant pas être trop importante pour que le matériau résultant reste globalement hydrophobe et n'absorbe pas trop d'eau afin d'éviter les problèmes spécifiques des matériaux hydrophiles.

Avantageusement, l'acrylate hydroxylé et le méthacrylate hydroxylé représentent ensemble préférentiellement entre 5 et 20% en masse du mélange, et plus préférentiellement autour de 16 à 17% du mélange.

La proportion relative de ces deux monomères hydroxylés l'un par rapport à l'autre peut varier selon les cas de 30 à 70% pour l'un et inversement pour l'autre en fonction de la température de transition vitreuse souhaitée.

Le mélange contient également des composés réticulants permettant d'obtenir à la polymérisation un réseau tridimensionnel et non des polymères linéaires. Pour obtenir un tel maillage, on ajoute des monomères difonctionnels de préférence aux trifonctionnels : préférentiellement un diacrylate de diol et un diméthacrylate de diol.

L'utilisation à la fois d'un diacrylate et d'un diméthacrylate permet d'obtenir un matériau final homogène dans lequel l'ensemble des monomères initiaux sont liés et ne se séparent pas en phases distinctes. En effet, le mélange initial de monomères contient des composés acrylates et des composés méthacrylates qui ne polymérisent pas à la même vitesse. Le mélange de ces deux réticulants permet de réticuler l'ensemble des monomères avec une répartition homogène des noeuds de réticulation dans le polymère résultant, le taux de branchement étant sensiblement constant tout au long de la polymérisation.

Ces composés réticulants comportent des fonctions éthoxy et éventuellement hydroxy de façon à ne pas augmenter la température de transition vitreuse du matériau final et simultanément à maintenir un niveau d'hydrophilie homogène avec le reste de la composition.

Le diacrylate de diol utilisé est préférentiellement le diacrylate de diéthylène glycol, le diacrylate de triéthylène glycol ou un diacrylate d'alkyldiol dont la chaîne alkyle comporte de 2 à 6 atomes de carbone.

Le diméthacrylate de diol utilisé peut être le diméthacrylate de diéthylène glycol ou un diméthacrylate d'alkyldiol dont la chaîne alkyle comporte de 2 à 6 atomes de carbone. Un diméthacrylate hydroxylé tel que le diacrylate de glycerol apporte une contribution supplémentaire à l'hydrophilie.

La quantité de réticulants doit être suffisante pour que, dans le polymère final, il ne reste pas trop de longues chaînes pendantes riches en alkylacrylate ou en arylalkoxy-acrylate qui augmentent le caractère collant du polymère.

En outre, plus le taux de réticulation est important et plus le temps de relaxation du polymère est court, la lentille se déployant plus vite dans l'oeil à température de transition vitreuse constante.

Mais d'autre part, on pourrait s'attendre à ce que le polymère résultant devienne cassant si son taux de réticulation est trop important. Ce phénomène est toutefois considérablement réduit par l'utilisation selon l'invention d'au moins un agent de transfert.

La quantité de diacryliques de diol et de diméthacryliques de diol est soigneusement choisie. De préférence, on ajoute au mélange ces composés réticulants en quantités telles que le niveau de réticulation final soit une masse entre noeuds de réticulation comprise entre 2000 gr/M et 10000 gr/M.

Avantageusement, l'ensemble diacrylate de diol et diméthacrylate de diol représente préférentiellement entre 1 et 3% en masse du mélange, la proportion relative entre le diacrylate de diol et le diméthacrylate de diol variant de préférence de 30 à 70% de l'un par rapport à l'autre et inversement.

En résumant les considérations détaillées ci-dessus, on peut imaginer un mélange particulier de monomères conduisant par polymérisation radicalaire à un mode de réalisation préférentiel du matériau selon l'invention.

Ce mélange comprenant préférentiellement au moins les monomères suivants : le 2-phénoxy-éthylacrylate, l'acrylate de butyle, l'acrylate d'hydroxy-éthyle ou l'acrylate d'hydroxybutyle, le méthacrylate d'hydroxy-éthyle, le diacrylate de diéthylène glycol, et le diméthacrylate de diéthylène ou de triéthylène glycol.

Le matériau selon l'invention n'est cependant pas limité aux monomères précédemment cités, d'autres monomères pouvant bien entendu être rajoutés au mélange, comme par exemple le diméthacrylate d'éthylène glycol ou le diméthacrylate de glycérol qui peuvent être ajoutés en plus du diacrylate de diéthylène glycol et du diméthacrylate de diéthylène ou de triéthylène glycol afin d'ajuster le niveau de réticulation.

D'une façon générale, les monomères choisis présentent préférentiellement une température d'ébullition supérieure à 100°C afin d'éviter les problèmes de bulles en fin de polymérisation à haute température, et une masse molaire comprise entre 130 et 350 g/mol qui permet d'éliminer facilement les monomères résiduels non polymérisés à la fin de la réaction de polymérisation.

Afin de rendre ce descriptif plus complet, on va maintenant décrire un exemple de procédé d'obtention d'un matériau polymère acrylique selon l'invention à partir du mélange initial préférentiel détaillé ci-dessus.

Pour réaliser la polymérisation recherchée, on commence par mélanger entre eux tous les différents monomères nécessaires à la réaction. Avantageusement, ces monomères sont solubles les uns dans les autres et une simple agitation suffit à réaliser un mélange homogène de ceux-ci.

On ajoute ensuite à ce mélange le composé ou les composés initiateurs nécessaires pour déclencher la réaction de polymérisation.

Lorsqu'il s'agit d'un peroxyde, celui-ci est généralement instable et peu soluble à température ambiante dans le mélange de monomères. Il est alors préférable de le dissoudre dans une fraction de mélange préalablement chauffée à une température d'environ 40°C, puis de remélanger cette fraction contenant le composé initiateur au reste du mélange de monomères.

L'agent de transfert est également rajouté au mélange avant ou après le composé initiateur.

Le mélange obtenu est conservé au réfrigérateur, à l'abri de la lumière.

Pour réaliser la polymérisation, on prélève de petites quantités de ce mélange que l'on place dans des moules et que l'on porte à une température comprise entre 75°C et 95°C.

Selon la température réactionnelle choisie, la polymérisation est plus ou moins rapide et plus ou moins efficace.

A 75°C par exemple, la polymérisation dure environ dix heures et le polymère obtenu est de grande qualité avec un faible taux de monomères résiduels, c'est-à-dire de monomères n'ayant pas réagi. Au contraire à 95°C, la polymérisation ne dure qu'une heure, mais le taux de monomères résiduels reste important.

Si l'on utilise un second peroxyde, on termine la polymérisation par une deuxième étape de cuisson à une température plus élevée, préférentiellement supérieure à la première de 15 à 20°C.

Pendant la polymérisation, la température de l'ensemble doit être surveillée de façon à être maintenue sensiblement constante. Les calories dégagées par la réaction qui est exothermique doivent être évacuées par exemple par convection aéraulique ou hydraulique.

Une fois la réaction terminée et après refroidissement, on procède au démoulage du polymère.

Les moules sont préférentiellement choisis de façon à obtenir après démoulage des blocs de polymère de forme générale cylindrique de faible hauteur, de type « jeton ou « palet ». Une telle forme est parfaitement adaptée à un usinage ultérieur de ces blocs de polymère en vue d'obtenir les lentilles intraoculaires.

Bien entendu, un moulage direct des lentilles intraoculaires est également possible avec un moule adapté.

Les blocs de polymère doivent ensuite être purifiés, afin de les débarrasser des monomères n'ayant pas réagi et des produits résiduels provenant notamment de la synthèse de chacun des monomères utilisés.

Pour cela, les blocs de polymère sont placés sous une atmosphère non oxydante, c'est-à-dire sous le flux d'un gaz non oxydant tel que de la vapeur d'eau, de l'argon ou de l'azote par exemple, à une température comprise entre 120°C et 150°C et sous une pression réduite comprise entre 20 et 100 mbars.

La durée de ce traitement est comprise entre 12 et 48 heures selon la température choisie.

Avec un tel traitement, on peut évaporer jusqu'à 4 à 10% de produits extractibles.

Les blocs de matériau polymère sont alors prêts à être usinés, à une température inférieure à la température de transition vitreuse du polymère, pour réaliser les lentilles intraoculaires selon l'invention.

Afin de parfaitement décrire l'invention, plusieurs exemples de matériaux polymères acryliques selon l'invention sont détaillés ci-dessous. Ces matériaux ont été obtenus par polymérisation radicalaire à partir des mélanges initiaux suivants : (Les quantités sont exprimées en pourcentages massiques.)

### Exemple 1 :

### MONOMÈRES :

- 2 phénoxy-éthylacrylate 78,4%
- acrylate de n-butyle 13,1 %
- acrylate d'hydroxy-éthyle 2,6%
- méthacrylate d'hydroxy-éthyle 4,4%
- diacrylate, de diéthylène glycol 0,5%
- diméthacrylate de diéthylène glycol 0,5%
- diméthacrylate d'éthylène glycol 0,5%

### COMPOSÉ INITIATEUR :

- diperoxyde de lauroyle 0,5%

### AGENT DE TRANSFERT:

- butane thiol 0,05%

On obtient ainsi, après polymérisation. à 85°C pendant une durée de quatre heures, un matériau polymère acrylique présentant un indice optique égal à 1,536 et une température de transition vitreuse sensiblement égale à -8,4°C.

### Exemple 2 :

### MONOMÈRES :

- 2 phénoxy-éthylacrylate 73,7%
- acrylate de n-butyle 8,2%
- acrylate d'hydroxy-éthyle 12,2%
- méthacrylate d'hydroxy-éthyle 4,1%
- diacrylate de diéthylène glycol 0,6%
- diméthacrylate de diéthylène glycol 0,6%
- diméthacrylate d'éthylène glycol 0,6%

### COMPOSÉ INITIATEUR :

- diperoxyde de lauroyle 0,5%

### AGENT DE TRANSFERT :

- butane thiol 0,05%

On obtient ainsi, après polymérisation à 75°C pendant une durée de neuf heures, un matériau polymère acrylique présentant un indice optique égal à 1,535 et une température de transition vitreuse sensiblement égale à -7,7°C.

### Exemple 3 :

### MONOMÈRES :

- 2 phénoxy-éthylacrylate 72,1%
- acrylate de n-butyle 10,3%
- acrylate d'hydroxy-éthyle 10,3%
- méthacrylate d'hydroxy-éthyle 5,2%
- diacrylate de diéthylène glycol 0,7%
- diméthacrytate de diéthylène glycol 0,7%
- diméthacrylate d'éthylène glycol 0,7%

### COMPOSÉ INITIATEUR :

- diperoxyde de lauroyle 0,5%

### AGENT DE TRANSFERT:

- butane thiol 0,05%

On obtient ainsi, après polymérisation à 75°C pendant une durée de neuf heures, un matériau polymère acrylique présentant un indice optique égal à environ 1,532 et une température de transition vitreuse sensiblement égale à -7,6°C.

### Exemple 4 :

### MONOMÈRES :

- 2 éthoxy-éthylméthacrylate 48%
- acrylate de butyle 37%
- méthacrylate d'hydroxy-éthyle 12%
- diméthacrylate d'éthylène glycol 1,9%

### COMPOSÉ INITIATEUR :

- peroxyde rapide 0,5%

### AGENT DE TRANSFERT :

- butane thiol 0,07%

On obtient ainsi, après polymérisation, un matériau polymère acrylique présentant un indice optique égal à environ 1,478.

### Exemple 5 :

### MONOMÈRES :

- 2 phénoxy-éthylacrylate 81%
- acrylate de 4-hydroxy-butyle 14,43%
- méthacrylate d'hydroxy-éthyle 2,5%
- diacrylate de diéthylène glycol 0,5%
- diméthacrylate de diéthylène glycol 1,2%

### COMPOSÉ INITIATEUR :

- peroxyde rapide 0,1%
- peroxyde lent 0,25%

### AGENT DE TRANSFERT:

- octane thiol 0,02%

On obtient ainsi, après polymérisation, un matériau polymère acrylique présentant un indice optique égal à environ 1,535.

## Revendications

1. Matériau polymère acrylique, hydrophobe et réticulé, à déformabilité augmentée, destiné à la réalisation d'implants ophtalmologiques et plus particulièrement de lentilles intraoculaires, matériau se présentant sous la forme d'un réseau macromoléculaire tridimensionnel obtenu par polymérisation radicalaire à partir d'un mélange de monomères acryliques ou d'un mélange de monomères acryliques et méthacryliques, **caractérisé en ce que** le mélange contient :
- des composés réticulants qui agissent pendant la polymérisation pour former le réseau tridimensionnel ; et en outre
- au moins un agent de transfert qui, lors de la polymérisation, interrompt localement la formation de ce réseau tridimensionnel pour créer des chaînes pendantes.

2. Matériau polymère acrylique selon la revendication précédente **caractérisé en ce que** l'agent de transfert est un thiol ou un produit halogéné.

3. Matériau polymère acrylique selon la revendication précédente **caractérisé en ce que** l'agent de transfert est un thiol contenant de quatre à dix-sept atomes de carbone ou un produit chloré.

4. Matériau polymère acrylique selon l'une quelconque des revendications précédentes **caractérisé en ce que** le mélange comprend entre 0,03 et 0,1% en masse d'agent de transfert.

5. Matériau polymère acrylique selon l'une quelconque des revendications précédentes **caractérisé en ce que** le mélange comprend en outre au moins un composé initiateur.

6. Matériau polymère acrylique selon la revendication précédente **caractérisé en ce que** le composé initiateur est un peroxyde d'alkyle.

7. Matériau polymère acrylique selon la revendication 5 **caractérisé en ce que** le mélange comprend entre 0,3 et 1% en masse de composé initiateur.

8. Matériau polymère acrylique selon l'une quelconque des revendications précédentes **caractérisé en ce que** le mélange comprend au moins les monomères suivants :
- un arylalcoxy-acrylate ou un arylalcoxy-méthacrylate ;
- un alkylacrylate ;
- un acrylate hydroxylé ;
- un méthacrylate hydroxylé ;
- un diacrylate de diol ; et
- un diméthacrylate de diol.

9. Matériau polymère acrylique selon la revendication précédente **caractérisé en ce que** l'arylalcoxy-acrylate ou l'arylalcoxy-méthacrylate est un composé choisi pour l'arylalcoxy-acrylate parmi le 2-phénoxy-éthylacrylate, le 2-phénoxy-2-éthoxy-éthylacrylate, le 2-phénoxy-2-éthoxy-2-éthoxy-éthylacrylate et ses oligomères supérieurs, et pour l'arylalcoxy-méthacrylate parmi le 2-phénoxy-éthylméthacrylate, le 2-phénoxy-2-éthoxy-éthylméthacrylate, le 2-phénoxy-2-éthoxy-2-éthoxy-éthylméthacrylate et ses oligomères supérieurs.

10. Matériau polymère acrylique selon la revendication 8 ou 9 **caractérisé en ce que** la chaîne alkyle de l'alkylacrylate comporte de 4 à 6 atomes de carbone.

11. Matériau polymère acrylique selon l'une quelconque des revendications 8 à 10 **caractérisé en ce que** l'acrylate hydroxylé est un monoacrylate de dihydroxy-alkyle ou de dihydroxy-éthoxy-alkyle dont la chaîne alkyle du glycol comporte de 2 à 6 atomes de carbone; et **en ce que** le méthacrylate hydroxylé est un monométhacrylate de dihydroxy-alkyle ou de dihydroxy-éthoxy-alkyle dont la chaîne alkyle du glycol comporte de 2 à 6 atomes de carbone.

12. Matériau polymère acrylique selon l'une quelconque des revendications 8 à 11 **caractérisé en ce que** le diacrylate de diol est le diacrylate de diéthylène glycol, le diacrylate de triéthylène glycol ou un diacrylate d'alkyldiol dont la chaîne alkyle comporte de 2 à 6 atomes de carbone ; et **en ce que** le diméthacrylate de diol est le diméthacrylate de diéthylène glycol ou un diméthacrylate d'alkyldiol dont la chaîne alkyle comporte de 2 à 6 atomes de carbone.

13. Matériau polymère acrylique selon la revendication 8 **caractérisé en ce qu'**il est obtenu par polymérisation radicalaire à partir d'un mélange comprenant au moins les monomères suivants :
- le 2-phénoxy-éthylacrylate ;
- l'acrylate de butyle ;
- l'acrylate d'hydroxy-éthyle ou l'acrylate d'hydroxybutyle ;
- le méthacrylate d'hydroxy-éthyle ;
- le diacrylate de diéthylène glycol ;
- le diméthacrylate de diéthylène glycol ou le diméthacrylate de triéthylène glycol.

14. Matériau polymère acrylique selon la revendication précédente **caractérisé en ce que** le mélange comprend en outre du diméthacrylate d'éthylène glycol ou du diméthacrylate de glycérol.

15. Matériau polymère acrylique selon la revendication 8 **caractérisé en ce que** le mélange comprend :
- entre 45 et 89% en masse d'arylalcoxy-acrylate ou d'arylalcoxy-méthacrylate ;
- entre 5 et 20% en masse d'alkylacrylate ;
- entre 5 et 20% en masse d'un mélange d'acrylate hydroxylé et de méthacrylate hydroxylé ;
- entre 1 et 3% en masse d'un mélange de diacrylate de diol et de diméthacrylate de diol.

16. Matériau polymère acrylique selon la revendication précédente **caractérisé en ce que** la proportion relative entre l'acrylate hydroxylé et le méthacrylate hydroxylé et entre le diacrylate de diol et le diméthacrytate de diol varie pour chaque couple de 30 à 70% de l'un par rapport à l'autre.

17. Lentille intraoculaire à implanter chirurgicalement dans le sac cristallinien, en remplacement du cristallin naturel, **caractérisé en ce qu'**elle est réalisée à partir d'un matériau polymère acrylique selon l'une quelconque des revendications précédentes.

18. Procédé de fabrication par polymérisation radicalaire, d'un matériau polymère acrylique, hydrophobe et réticulé, à déformabilité augmentée, destiné à la réalisation d'implants ophtalmologiques et plus particulièrement de lentilles intraoculaires, procédé **caractérisé en ce que** :
- l'on réalise un mélange contenant des monomères acryliques ou des monomères acryliques et méthacryliques, des composés réticulants et au moins un agent de transfert ; et
- l'on polymérise ce mélange, de manière à obtenir par cette polymérisation un réseau macromoléculaire tridimensionnel qui comporte localement des chaînes pendantes.

## Claims

1. A cross-linked, hydrophobic, acrylic polymer material, with improved deformability, intended for the production of ophthalmological implants and more particularly of intraocular lenses, material which is a three-dimensional macromolecular network obtained by radical polymerization starting from a mixture of acrylic monomers or a mixture of acrylic and methacrylic monomers, **characterized in that** the mixture comprises:
- cross-linking compounds that act during the polymerization to form the three-dimensional network; and additionally
- at least a transfer agent that, when the polymerization occurs, interrupts locally the three-dimensional network formation to create dangling chains.

2. An acrylic polymer material according to the preceding claim, **characterized in that** the transfer agent is a thiol or a halogenated product.

3. An acrylic polymer material according to the preceding claim, **characterized in that** the transfer agent is a thiol containing four to seventeen carbon atoms or a chlorinated product.

4. An acrylic polymer material according to any one of the preceding claims, **characterized in that** the mixture comprises between 0.03 and 0.1 % by weight of transfer agent.

5. An acrylic polymer material according to any one of the preceding claims, **characterized in that** the mixture additionally comprises at least one initiator compound.

6. An acrylic polymer material according to the preceding claim, **characterized in that** the initiator compound is an alkyl peroxide.

7. An acrylic polymer material according to claim 5, **characterized in that** the mixture comprises between 0.3 and 1% by weight of initiator compound.

8. An acrylic polymer material according to any one of the preceding claims, **characterized in that** the mixture comprises at least the following monomers:
- an arylalkoxy acrylate or an arylalkoxy methacrylate;
- an alkyl acrylate;
- a hydroxylated acrylate;
- a hydroxylated methacrylate;
- a diol diacrylate; and
- a diol dimethacrylate.

9. An acrylic polymer material according to the preceding claim, **characterized in that** the arylalkoxy acrylate or the arylalkoxy methacrylate is a compound chosen from among 2-phenoxyethyl acrylate, 2-phenoxy-2-ethoxyethyl acrylate, 2-phenoxy-2-ethoxy-2-ethoxyethyl acrylate and its higher oligomers for the arylalkoxy acrylate and from among 2-phenoxyethyl methacrylate, 2-phenoxy-2-ethoxyethyl methacrylate, 2-phenoxy-2-ethoxy-2-ethoxyethyl methacrylate and its higher oligomers for the arylalkoxy methacrylate.

10. An acrylic polymer material according to claim 8 or 9, **characterized in that** the alkyl chain of the alkyl acrylate contains 4 to 6 carbon atoms.

11. An acrylic polymer material according to any one of claims 8 to 10, **characterized in that** the hydroxylated acrylate is a dihydroxyalkyl or dihydroxy-ethoxyalkyl monoacrylate, wherein the alkyl chain of the glycol contains 2 to 6 carbon atoms; and **in that** the hydroxylated methacrylate is a dihydroxyalkyl or dihydroxy-ethoxyalkyl monomethacrylate, wherein the alkyl chain of the glycol contains 2 to 6 carbon atoms.

12. An acrylic polymer material according to any one of claims 8 to 11, **characterized in that** the diol diacrylate is diethylene glycol diacrylate, triethylene glycol diacrylate or an alkyldiol diacrylate, wherein the alkyl chain contains 2 to 6 carbon atoms; and **in that** the diol dimethacrylate is diethylene glycol dimethacrylate or an alkyldiol dimethacrylate, wherein the alkyl chain contains 2 to 6 carbon atoms.

13. An acrylic polymer material according to claim 8, **characterized in that** it is obtained by radical polymerization starting from a mixture comprising at least the following monomers:
- 2-phenoxyethyl acrylate;
- butyl acrylate;
- hydroxyethyl acrylate or hydroxybutyl acrylate;
- hydroxyethyl methacrylate;
- diethylene glycol diacrylate;
- diethylene glycol dimethacrylate or triethylene glycol dimethacrylate.

14. An acrylic polymer material according to the preceding claim, **characterized in that** the mixture additionally contains ethylene glycol dimethacrylate or glycerol dimethacrylate.

15. An acrylic polymer material according to claim 8, **characterized in that** the mixture comprises:
- between 45 and 89% by weight of arylalkoxy acrylate or arylalkoxy methacrylate;
- between 5 and 20% by weight of alkyl acrylate;
- between 5 and 20% by weight of a mixture of hydroxylated acrylate and hydroxylated methacrylate;
- between 1 and 3% by weight of a mixture of diol diacrylate and diol dimethacrylate.

16. An acrylic polymer material according to the preceding claim, **characterized in that** the relative proportion between the hydroxylated acrylate and the hydroxylated methacrylate and between the diol diacrylate and the diol dimethacrylate varies from 30 to 70% of the one relative to the other, for each pair.

17. An intraocular lens to be implanted surgically in the crystalline lens capsule, replacing the natural crystalline lens, **characterized in that** it is produced starting from an acrylic polymer material according to any one of the preceding claims.

18. A method for manufacturing by radical polymerization a cross-linked, hydrophobic, acrylic polymer material, with improved deformability, intended for production of ophthalmological implants and more particularly of intraocular lenses, **characterized in that**:
- a mixture comprising acrylic monomers or acrylic and methacrylic monomers, cross-linking compounds and at least a transfer agent, is realized; and
- said mixture is polymerized, in order to obtain by this polymerization a three-dimensional macromolecular network that locally comprises dangling chains.

## Patentansprüche

1. Hydrophobes vernetztes Acrylpolymermaterial mit erhöhter Verformbarkeit für die Herstellung von ophthalmologischen Transplantaten, genauer gesagt von intraokulären Linsen, Material in Form eines dreidimensionalen makromolekularen Netzes, erhalten durch radikalische Polymerisation ausgehend von einer Mischung von Acrylmonomeren oder von einer Mischung von Acryl- und Methacrylmonomeren, **gekennzeichnet dadurch, dass** die Mischung folgendes enthält :
- vernetzende Verbindungen, die während die Polymerisation die Bildung des dreidimensionalen Netzes bewirken; und außerdem
- mindestens ein Übertragungsmittel, das bei der Polymerisation örtlich die Bildung dieses dreidimensionalen Netzes unterbricht, um hängende Ketten zu schaffen.

2. Acrylpolymermaterial nach dem vorherigen Anspruch, **gekennzeichnet dadurch, dass** das Übertragungsmittel ein Thiol oder ein halogeniertes Produkt ist.

3. Acrylpolymermaterial nach dem vorherigen Anspruch, **gekennzeichnet dadurch, dass** das Übertragungsmittel ein Thiol ist, das vier bis siebzehn Kohlenstoffatome enthält, oder ein chloriertes Produkt.

4. Acrylpolymermaterial nach einem beliebigen der vorherigen Ansprüche, **gekennzeichnet dadurch, dass** die Mischung zwischen 0,03 und 0,1% Übertragungsmittelmasse umfasst.

5. Acrylpolymermaterial nach einem beliebigen der vorherigen Ansprüche, **gekennzeichnet dadurch, dass** die Mischung außerdem mindestens eine Initiatorverbindung umfasst.

6. Acrylpolymermaterial nach dem vorherigen Anspruch, **gekennzeichnet dadurch, dass** die Initiatorverbindung ein Alkylperoxid ist.

7. Acrylpolymermaterial nach Anspruch 5, **gekennzeichnet dadurch, dass** die Mischung zwischen 0,3 und 1% Initiatorverbindungsmasse umfasst.

8. Acrylpolymermaterial nach einem beliebigen der vorherigen Ansprüche, **gekennzeichnet dadurch, dass** die Mischung mindestens die folgender, Monomere umfasst:
- ein Arylalcoxy-acrylat oder ein Arylalcoxy-methacrylat;
- ein Alkylacrylat;
- ein hydroxyliertes Akrylat;
- ein hydroxyliertes Methacrylat;
- ein Dioldiacrylat; und
- ein Dioldimethacrylat.

9. Acrylpolymermaterial nach dem vorherigen Anspruch, **gekennzeichnet dadurch, dass** das Arylalcoxyacrylat oder das Arylalcoxymethacrylat eine Verbindung ist, die aus folgenden ausgewählt ist: für das Arylalcoxyacrylat, aus 2-phenoxyethylacrylat, 2-phenoxy-2-ethoxyethylacrylat, 2-phenoxy-2-ethoxy-2-ethoxyethylacrylat und seinen höheren Oligomeren, und für das Arylalcoxymethacrylat aus 2-phenoxyethylmethacrylat, 2-phenoxy2-ethoxyethylmethacrylat, 2-phenoxy-2-ethoxy-2-ethoxyethylmethacrylat und seinen höheren Oligomeren.

10. Acrylpolymermaterial nach Anspruch 8 oder 9, **gekennzeichnet dadurch, dass** die Alkylkette des Alkylacrylats 4 bis 6 Kohlenstoffatome aufweist.

11. Acrylpolymermaterial nach einem beliebigen der Patentansprüche 8 bis 10, **gekennzeichnet dadurch, dass** das hydroxylierte Akrylat ein Monoacrylat von Dihydroxyalkyl oder von Dihydroxyethoxyalkyl ist, dessen Alkylkette des Glykols 2 bis 6 Kohlenstoffatome aufweist; sowie dadurch, dass das hydroxylierte Methacrylat ein Monomethacrylat von Dihydroxyalkyl oder von Dihydroxyethoxyalkyl ist, dessen Alkylkette des Glykols 2 bis 6 Kohlenstoffatome aufweist.

12. Acrylpolymermaterial nach einem beliebigen der Patentansprüche 8 bis 11, gekennzeichnet dadurc,h dass das Dioldiacrylat das Diacrylat von Diethylenglykol ist, das Diacrylat von Triethylenglykol oder ein Alkyldioldiacrylat, dessen Alkylkette 2 bis 6 Kohlenstoffatome aufweist; sowie dadurch, dass das Dioldimethacrylat das Dimethacrylat von Diethylenglykol ist oder ein Alkyldioldimethacrylat, dessen Alkylkette 2 bis 6 Kohlenstoffatome aufweist.

13. Acrylpolymermaterial nach Anspruch 8, **gekennzeichnet dadurch, dass** es durch radikalische Polymerisation erhalten wird, ausgehend von einer Mischung mit mindestens den folgenden Monomeren :
- 2-phenoxyethylacrylat;
- Butylakrylat;
- Hydroxyethylakrylat oder Hydroxybutylakrylat;
- Hydroxyethylmethacrylat;
- Diethylenglykoldiacrylat;
- Diethylenglykoldimethacrylat oder Triethylenglykoldimethacrylat.

14. Acrylpolymermaterial nach dem vorherigen Anspruch, **gekennzeichnet dadurch, dass** die Mischung außerdem Äthylenglykoldimethacrylat oder Glyceroldimethacrylat umfasst.

15. Acrylpolymermaterial nach Anspruch 8, **gekennzeichnet dadurch, dass** die Mischung folgendes umfasst:
- zwischen 45 und 89% Arylalcoxyacrylat- oder Arylalcoxymethacrylatmasse;
- zwischen 5 und 20% Alkylacrylatmasse;
- zwischen 5 und 20% Masse einer Mischung aus hydroxyliertem Akrylat und hydroxyliertem Methacrylat;
- zwischen 1 und 3% Masse einer Mischung von Dioldiacrylat und Dioldimethacrylat.

16. Acrylpolymermaterial nach dem vorherigen Anspruch, **dadurch gekennzeichnet, dass** der relative Verhältnis zwischen dem hydroxylierten Akrylat und dem hydroxylierten Methacrylat und zwischen dem Dioldiacrylat und dem Dioldimethacrylat für jede Paars 30 bis 70% voneinander variiert.

17. Chirurgisch in die Linsenkapsel zu implantierende intraokuläre Linse, als Ersatz der natürlichen Linse, **dadurch gekennzeichnet, dass** sie ausgehend von dem Acrylpolymermaterial nach einem beliebigen der vorherigen Ansprüche verwirklicht wird.

18. Herstellungsverfahren für ein Acrylpolymermaterial, hydrophob und vernetzt, mit erhöhter Verformbarkeit, durch radikalische Polymerisation für die Herstellung von ophthalmologischen Transplantaten, genauer gesagt von intraokulären Linsen, **gekennzeichnet dadurch, dass**:
- man eine Mischung herstellt, die Acrylmonomere enthält oder Acryl-und Methacrylmonomere, vernetzende Verbindungen und mindestens ein Übertragungsmittel; und
- man diese Mischung polymerisiert, um durch diese Polymerisation ein dreidimensionales makromolekulares Netz zu erhalten, das örtlich hängende Ketten aufweist.
